# EUROPEAN PATENT APPLICATION

(11) **EP 0 670 166 A2**
(43) Date of publication of application: **06.09.1995**
(21) Application number: 95102558.4
(22) Date of filing: 23.02.1995
(51) Int. Cl.: A61K 47/44, A61K 47/12, A61K 38/15, A61K 9/48

(54) **Formulations for orally administered pharmaceutical agents**

(30) Priority: 01.03.1994 US 203424
(71) Applicant: ELI LILLY AND COMPANY, Indianapolis, Indiana 46285 (US)
(72) Inventor: Sweetana, Stephanie A., Bloomington, Indiana 46285 (US)
(74) Representative: Crowther, Terence Roger

(57) **Abstract**

The present invention provides a novel formulation for the delivery of pharmaceutical compounds through oral administration, which allows for substantial bioavailability of the active ingredient of the formulation. These formulations comprise a pharmaceutical compound that is substantially insoluble in water. This compound is solubilized in a carrier which comprises a long chain free fatty acid solubilizing agent, a pharmaceutically acceptable oil and a surface active agent. Through this formulation, the solubility of the pharmaceutical compound is increased and, thereby, systemic delivery is improved.

## Description

### BACKGROUND OF THE INVENTION

### 1. Field of the Invention

This invention relates generally to oral delivery formulations of pharmaceutical agents and compounds.

### 2. Description of the Prior Art

Historically, the oral route of administration has been the most preferred for chronic drug therapy. For certain pharmaceutical agents, however, oral administration results in only small amounts of active ingredient reaching the bloodstream. This is typically due to enzymatic degradation in the stomach and small intestine, and poor uptake from the intestinal tract. Attempts have been made to increase the intestinal absorption by use of formulations that protect the drugs from enzymatic degradation and/or enhance the uptake from the intestinal tract.

International Application No. JP89/00748 teaches an enteric formulation with physiologically active proteins or peptide, in which a solid mass of a proteinaceous drug is mixed with a nonionic surfactant and a pharmaceutically acceptable enteric material capable of dissolving in the duodenal juice and also in an organic volatile solvent. The preferred enteric material is a polymeric material, used for forming an enteric coating on tablets, and the like.

European Patent Application 92302487.1 teaches solid pharmaceutical formulation for Aureobasidins, which includes a surface active agent, a coating agent, a viscosity agent, a filler, and other agents, such as stabilizers, coloring agents, fragrance agents, etc. The application indicates that oral administration of this formulation results in a concentration of Aureobasidin in the blood high enough to yield a pharmaceutical affect, such as antifungal activity.

Stella, et al. *J. Pharm. Sci.* Vol. 67, No. 10, p. 1375 (1978), teach the bioavailability of a hydrophobic amine antimalarial compound by formulation with oleic acid in soft gelatin capsules. Because of the extremely low solubility of the antimalarial compound, there is poor bioavailability; therefore, a large dosage of the unformulated drug is required to achieve effective blood levels. Stella, et al. report promising results relating to a formulation of the antimalarial compound, using oleic acid as a solvent in the soft gel capsules.

Similarly, Tokumura, et al., ,*J. Pharm. Sci.*, Vol. 76, No. 4, p. 286 (April 1987), teach that the bioavailability of cinnarizine is enhanced by solubilization in oleic acid and linoleic acid. Tokumura, et al. postulated that when cinnarizine was administered as an oleic acid solution, that a mixed micelle of cinnarizine with oleic acid and bile salts was formed in the gastrointestinal tract. Through this micelle formation, the absorption of the cinnarizine was increased.

Kararli, et al., *Pharm. Res.* Vol. 9, No. 7, p. 888 (1992) teach the oral delivery of a renin inhibitor compound having low bioavailability, using emulsion formulations. In an attempt to increase the bioavailability, the renin inhibitor was dissolved in an oil and an oil water emulsion was formed, using oleic acid and glycerides. The use of this emulsion when mixed in a bile salt system, increased the solubility of the renin inhibitor.

U.S. Patent No. 4,388,307 teaches cyclosporin formulations, including a carrier comprising a product of a natural hydrogenated vegetable oil triglyceride and a polyalkylene and polyol, a saturated fatty acid triglyceride, and a mono- or diglyceride. The '307 patent further teaches the use of ethanol as a solubilizing agent and a vegetable oil as a carrier. It is suggested that such a formulation will increase the bioavailability and absorption of the cyclosporin.

While this prior art shows some promising results, therapeutic doses of many pharmaceutical agents cannot be practically and effectively delivered orally, utilizing the prior art.

### SUMMARY OF THE INVENTION

It is the object of the present invention to provide a novel formulation for the delivery of pharmaceutical compounds through oral administration, which allows for substantial bioavailability of the active ingredient of the formulation. These formulations comprise a pharmaceutical compound that is substantially insoluble in water. This compound is solubilized in a carrier which comprises a long chain free fatty acid solubilizing agent, a pharmaceutically acceptable oil and a surface active agent. Through this formulation, the solubility of the pharmaceutical compound is increased and, thereby, systemic delivery is improved.

It is a further object of the present invention to provide a formulation which is particularly applicable to cyclicpeptide compounds which are substantially insoluble in water and poorly absorbed.

It is another object of the present invention to provide a general oral formulation discussed above, in which the characteristic of the constituents can be adjusted to best suit the active pharmaceutical compound. The characteristics of the long chain free fatty acid solubilizing agent may be varied and adjusted with respect to the particular pharmaceutical compound contained in the formulation. Specifically, the degree of unsaturation and the chain length of the free fatty acid can be varied. The pharmaceutically acceptable oil may also be varied somewhat in fatty acid composition and in chain length to enhance the solubility. Slight variations of other excipients may also have advantageous qualities.

It is a yet further object of the present invention to provide an oral formulation that solubilizes the antifungal compound aureobasidin A, and upon oral administration produces high plasma levels in animals, relative to other oral formulations. This formulation provides a favorable pharmaco-kinetic profile which correlates with improved efficacy of the compound toward infections. The formulation is preferably administered as (1) a liquid, (2) a liquid admixed or diluted with a suitable aqueous vehicle to form an emulsion, (3) an encapsulated compound, or (4) a lipid absorbed in a solid matrix.

Still a further object of the present invention is to provide an oral formulation that delivers an effective amount of the active ingredient without an objectionable taste.

The advantages of use of the formulations of the present invention are many. Most importantly, it provides a formulation which delivers a efficacious amount of the active ingredient to the bloodstream of an animal. Because the active ingredients are not generally soluble in aqueous solutions, such delivery has been difficult in the past.

Yet another advantage of the present invention is that it provides an effective method of delivering an active ingredient to a patient. Because the formulation is provided for oral administration, it can be taken by a patient, without the need for parenteral administration. Patient compliance is thereby increased.

Still another advantage of the present invention is to provide a formulation which can be easily formulated from existing materials.

Thus, the present invention provides novel formulations of antifungal agents and other compounds that are practical and effective.

Further objects and advantages of the invention will be apparent upon reviewing the description of the preferred embodiment and claims of the invention.

### DETAILED DESCRIPTION OF THE PREFERRED EMBODIMENT

As indicated above, materials substantially insoluble in water have been very difficult to deliver to the bloodstream of an animal, through oral administration. Applicant has herein developed formulations which enhance such delivery. Surprisingly, the combination of known ingredients in the correct ratio supplies enhanced bioavailability of substantially insoluble materials.

In general, the formulations of the present invention relate to a compound substantially insoluble in water, which is solubilized in an oil based carrier. This carrier includes at least three elements: 1) a long chain free fatty agent solubilizing agent; 2) a pharmaceutically acceptable oil; and 3) a surface active agent.

In a preferred embodiment, the long chain free fatty acid solubilizing agent is comprised of oleic acid. While oleic acid is the preferred solubilizing agent, other free fatty acids can be used. Preferably, these free fatty acids have as a chain length between C12 to C22. Such free fatty acids include linoleic acid and linolenic acid. A preferred characteristic of the long chain free fatty acid solubilizing agent is that it should be soluble in a triglyceride carrier. In a particularly preferred embodiment, solubilizing agent component is about 5% to 95% by volume of the oral formulation.

The pharmaceutically acceptable oil of the present invention is preferably composed of long chain fatty acids C6 to C22 in length, and comprises about 5% to 95% by volume of the formulation. More preferably, the long chain fatty acids have a chain length of C12 to C20. In a particularly preferred embodiment the pharmaceutically acceptable oil is a triglyceride. Examples of such triglycerides are corn oil, olive oil, soya oil, peanut oil and sunflower oil. In an especially preferred embodiment, corn oil is used as the pharmaceutically acceptable oil.

The formulation also includes a surface active ingredient. Appropriate surface active agents include both ionic and non-ionic surfactants. The surface active agent is preferably a nonionic surfactant that is soluble in a triglyceride. Such surfactants include polyglycolized glycerides, sorbitan fatty acid esters, poloxamers, polyoxyethylene sorbitan fatty acid esters, polyoxyethylene (POE) derivatives, ethoxylated fatty esters, mono-glycerides and ethoxylated derivatives thereof, diglycerides and polyoxyethylene derivatives thereof. Suitable ionic surfactants include sodium lauryl sulfate, docusate sodium, cholic acid and salts and derivatives thereof, lecithins and phospholipids. In a particularly preferred embodiment, the surfactant is Peglicol 5 Oleate, sold under the trade name Labrafil® M1994CS. The surfactant preferably comprises between 0.1% to 50% weight percent of the formulation.

While the present invention is effective in delivering many pharmaceuticals, in a preferred embodiment, it is directed to antifungal compounds, specifically, the antifungal compound aureobasidin A. Aureobasidin A is also known as NK204 (Nippon Kayaku) and R106-I (Takara Shuza). Synthesis of aureobasidin A is described in U.S. Patent No. 5,057,493 and EPA 92302487.1.

An example of a preferred final formulation of the present invention where the pharmaceutical agent is aureobasidin A has the following composition:
Aureobasidin A at about 17.5% w/v;
oleic acid at about 17.1% v/v;
Labrafil® M1944CS at about 0.43% w/v; and
corn oil at about 68% v/v.

### EXAMPLE 1

The oral formulations of pharmaceutical compounds are manufactured by the following process.

Liquid excipients are measured by either weight or volume. Volume measurements may be converted to weight measurements by determination of the specific gravity of the components, prior to use. The surfactant, the long chain fatty acid solubilizing agent and the pharmaceutically acceptable oil are combined and thoroughly mixed by a simple propeller mixer, such as a Lightnin® mixer. While mechanically stirring the combination, the pharmaceutical compound is gradually added to the excipient blend and mixed until solution is complete. The necessary volume or weight of the drug is filled into glass bottles. Filling may be done by hand, using a suitable pipette or with automated filling equipment.

### EXAMPLE 2

One ml of an aureobasidin A oral solutions are prepared using the method from EXAMPLE 1, with the following excipients.

| **Ingredient** | **Dose** | |
|---|---|---|
| | **25 mg/mL** | **175 mg/mL** |
| Aureobasidin A | 25.000 mg | 175.000 mg |
| Peglicol 5 Oleate | 4.897 mg | 4.280 mg |
| Oleic Acid, Food Grade | 0.196mL | 0.171 mL |
| Corn Oil, NF | 0.778 mL | 0.680 mL |

### EXAMPLES 3-4

The pharmacokinetic parameters and bioavailability of formulations of aureobasidin A were tested. A 20 mg/kg (weight of drug/patient body weight) dose of aureobasidin A was administered to female beagle dogs in an oral delivery formulation of the present invention. An aureobasidin A formulation in linoleic acid alone was also tested for comparison. Blood samples were drawn from the dogs and the concentration of aureobasidin A in the blood was measured by HPLC. This study yielded the following results.

| **Formulation** | **Dose** (mg/kg) | **Route** | **AUC**_{**0-∞**} (µg* hr/ml) | **Bioavailability** (%) |
|---|---|---|---|---|
| EXAMPLE 3 | | | | |
| Formulation Labrafil® Oleic Acid Corn Oil | 20 | Oral | 11.205±1.430 | 28.35 |

| EXAMPLE 4 | | | | |
|---|---|---|---|---|
| Formulation Linoleic Acid | 20 | Oral | 1.667±0.061 | 4.22 |

Preferably, the formulations of the present invention are administered as a liquid. However, other forms of the formulation are also contemplated by the invention. Such forms may include emulsions, encapsulation or the formulation being adsorbed onto a solid.

Emulsions may be formed by admixing or diluting the liquid formulations discussed above with an aqueous based diluent. In some circumstances, emulsions may have the beneficial effect of further enhancing the bioavailability of the pharmaceutical agent. Emulsions further provide the benefit of allowing the diluent to be flavored to make the formulation more palatable.

The formulations may also be encapsulated. The preferred method of encapsulation is filling soft gelatin capsules with the liquid formulations discussed above. Such filling methods are well known in the art. It is also contemplated the present invention may be microencapsulated. Methods of microen-capsulation such as spray drying, lyophilization or emulsion drying are also well known in the art and applicable to the present invention.

In addition, adsorption onto a solid matrix provides a further form of the present invention. Such adsorbed solids may have the advantage of providing a sustained delivery system. See, Berthod, et al., "Dry Adsorbed Emulsions: An Oral Sustained Delivery System," *J. Pharm. Sci.*, Vol. 77, No. 3, p. 216 (1988).

The above description and examples are meant only to be representative of the present invention, and are, by no means, limiting. Only the following claims limit the present invention.

## Claims

1. A substantially non-aqueous formulation for oral administration of a pharmaceutical agent comprising:
(1) a pharmaceutical agent that is substantially insoluble in water; and
(2) a substantially non-aqueous carrier in which said agent is solubilized, said carrier comprising a long chain fatty acid solubilizing agent, a pharmaceutically acceptable oil and a surface active agent.

2. The formulation of Claim 1 wherein the solubilizing agent is about 5% to about 95% of the carrier.

3. The formulation of Claim 1 wherein the pharmaceutically acceptable oil is about 5% to about 95% of the carrier.

4. The formulation of Claim 1 wherein the surface active agent is about .01% to about 50% of the carrier.

5. The formulation of Claim 1 wherein the pharmaceutical agent is a cyclicpeptide.

6. The formulation of Claim 5 wherein the cyclicpeptide is an antifungal agent.

7. The formulation of Claim 6 wherein the antifungal agent is aureobasidin A.

8. The formulation of Claim 1 wherein the long chain fatty acid solubilizing agent is selected from the group of oleic acid, linoleic acid and linolenic acid; the pharmaceutically acceptable oil is selected from the group consisting of corn oil, olive oil, peanut oil, soybean oil, sunflower oil and mixtures thereof; and the surface active agent is selected from the group of polyglycolized glycerides, sorbitan fatty acid esters, poloxamers, polyoxyethylene sorbitan fatty acid esters, polyoxyethylene (POE) derivatives, ethoxylated fatty esters, mono-glycerides and ethoxylated derivatives thereof, diglycerides and polyoxyethylene derivatives thereof, sodium lauryl sulfate, docusate sodium, cholic acid and salts and derivatives thereof, lecithins and phospholipids.

9. The formulation of Claim 1 wherein the solubilizing agent is a free fatty acid with chain length from C12 to C22 which is soluble in a triglyceride and is selected from the group of oleic acid, linoleic acid, linolenic acid or mixtures thereof.

10. The formulation of Claim 1 wherein the surface active agent is a non-ionic surfactant that is soluble in the triglyceride and is selected from the group of polyglycolized glycerides, sorbitan fatty acid esters, poloxamers, polyoxyethylene sorbitan fatty acid esters, polyoxyethylene (POE) derivatives, ethoxylated fatty esters, mono-glycerides and ethoxylated derivatives thereof, diglycerides and polyoxyethylene derivatives thereof.

11. The formulation of Claim 1 wherein the solubilizing agent comprises a free fatty acid with chain length from C12 to C22.

12. The formulation of Claim 11 wherein the solubilizing agent is soluble in a triglyceride.

13. The formulation of Claim 12 wherein the solubilizing agent comprises oleic acid.

14. The formulation of Claim 1 wherein the pharmaceutically acceptable oil has a chain length of C6 to C20.

15. The formulation of Claim 14 where in the pharmaceutically acceptable oil comprises a triglyceride.

16. The formulation of Claim 14 where in the pharmaceutically acceptable oil comprises corn oil.

17. The formulation of Claim 1 wherein the surface active agent comprises a non-ionic surfactant.

18. The formulation of Claim 17 wherein the surface active agent comprises peglicol 5 oleate.

19. The formulation of Claim 1 wherein the formulation is a liquid.

20. The formulation of Claim 1 wherein the formulation is encapsulated.

21. The formulation of Claim 20 wherein the encapsulation is performed by filling a liquid formulation into a gelatin capsule.

22. The substantially non-aqueous antifungal formulation for oral administration of Claim 1, comprising:
(1) 15% to 20% of the pharmaceutical agent, said pharamaceutical agent being aureobasidin A;
(2) 15% to 20% of the solubilizing agent, said solubilizing agent being oleic acid;
(3) 65% to 71% of the pharmaceutically acceptable oil, said oil being corn oil; and
(4) 0.1% to 5% of the surface active agent, said agent being peglicol 5 oleate.
